# EUROPÄISCHE PATENTSCHRIFT

(11) **EP 2 630 983 B1**
(45) Veröffentlichungstag und Bekanntmachung des Hinweises auf die Patenterteilung: **26.10.2016**
(21) Anmeldenummer: 12156560.0
(22) Anmeldetag: 22.02.2012
(51) Int. Cl.: A61M 39/10, A61B 18/02, F16L 37/56, F16L 39/04, A61M 39/12, A61M 39/20

(54) **Fluidkupplung mit mehreren radial beweglichen Steckelementen**
Fluid connector with multiple radially shiftable connector elements
Connecteur à fiches fluidique doté de plusieurs éléments mobiles de manière radiale

(43) Veröffentlichungstag der Anmeldung: 28.08.2013
(73) Patentinhaber: ERBE Elektromedizin GmbH, 72070 Tübingen (DE)
(72) Erfinder: Groß, Stefan, 72072 Tübingen (DE)
(74) Vertreter: Rüger, Barthelt & Abel

(56) Entgegenhaltungen:
- WO-A1-2007/118334
- WO-A1-2008/142672
- GB-A- 2 419 647
- US-A- 2 086 424
- US-A- 3 214 195
- US-A- 3 337 181
- US-A- 3 508 580
- US-A- 3 937 496
- US-A1- 2010 181 764

## Beschreibung

Die Erfindung betrifft ein Fluidsteckverbinderteil mit Steckelementen zur Aufnahme oder Abgabe von Fluiden, insbesondere für medizinische Geräte oder Instrumente.

In der Medizin werden sehr viele Instrumente eingesetzt, die zu ihrem Betrieb unterschiedliche gasförmige oder flüssige, d.h. fluide Medien benötigen, beispielsweise mehr oder weniger komprimierte Gase, Wasser oder dergleichen. Zur Speisung der Instrumente werden diese über einen Fluidsteckverbinder an ein entsprechendes medizinisches Gerät angeschlossen, das die Fluide in dem gewünschten Zustand und in gewünschter Menge bereitstellt. Die Fluide können dabei unter erheblichem Druck stehen, was z.B. bei kryochirurgischen Anwendungen der Fall ist. In einem noch relativ einfachen Fall muss dem Instrument das Kryofluid unter entsprechendem Druck zugeführt und das entspannte Kryofluid wieder abgeführt werden. Dazu sind zwei getrennte Fluidleitungen erforderlich. In vielen Fällen ist mindestens eine dritte Fluidleitung zur weiteren Rückführung des Kryofluids erforderlich, beispielsweise wenn der Gefriervorgang abgebrochen und der Auftauvorgang beschleunigt werden soll. Es können im Einzelfall weitere Fluidleitungen für im Behandlungsprozess erforderliche Fluide hinzukommen.

Es muss sichergestellt werden, dass die dazu verwendeten Fluidsteckverbinder leicht zu betätigen und zuverlässig dicht sind.

Aus der US 3,214,195 ist ein Fluidsteckverbinderteil bekannt, das mehrere Steckelemente aufweist, die eine gemeinsame Steckrichtung festlegen. Dem Steckelement ist ein Buchsenelement zugeordnet. Das Steckelement und das Buchsenelement ist jeweils quer zur Steckrichtung beweglich gelagert. Das beweglich gelagerte Steckelement und das beweglich gelagerte Buchsenelement sind jeweils von einem nicht gasbeaufschlagten Gehäuseteil umgeben. Das Buchsenelement enthält ein Dichtungselement zur Abdichtung und Zentrierung.

Aus der US 3,337,181 ist ein Fluidsteckverbinderteil mit zwei Fluidkanälen bekannt, wobei ein Steckerelement quer zur Steckrichtung bzw. radial beweglich gelagert ist. Damit sollen Fertigungstoleranzen ausgeglichen werden.

Weiter ist aus der GB 2 419 647 A ein Fluidsteckverbinderteil mit zwei Fluidkanälen bekannt. Das Steckerteil 2 ist durch hochflexible Elemente radial beweglich gehalten.

Die WO 2008/142672 A1 offenbart ein Fluidsteckverbinderteil mit einer Buchse, die quer zur Einsteckrichtung eines Steckers beweglich gelagert ist. Die Buchse 33 bildet ein Dichtungselement und ist insoweit etwas federnd ausgebildet.

Die WO 2007/118334 A1 offenbart ein Fluidsteckverbinderteil mit mehreren Steckstiften, die quer beweglich gelagert sind, um Fertigungstoleranzen auszugleichen. Gleiches gilt im Hinblick auf die US 2010/0181764 A1.

Die US 3,937,496 und die US 3,508,580 offenbaren Fluidstecker mit radial beweglich bzw. federnd gelagerten Stiften oder Buchsen. Außerdem offenbart die US 2,086,424 einen elektrischen Stecker mit quer zur Steckrichtung beweglichen Steckelementen.

Aufgabe der Erfindung ist es, ein Steckverbinderteil zu schaffen, mit dem sich mehrere Fluidkanäle zwischen einem medizinischen Gerät und einem medizinischen Instrument verbinden lassen, wobei wenigstens einer der oben genannten Anforderungen Rechnung getragen wird.

Diese Aufgabe wird mit dem Steckverbinderteil nach Anspruch 1 gelöst:

Das erfindungsgemäße Fluidsteckverbinderteil weist mindestens zwei Steckelemente auf, die jeweils als Steckbuchse ausgebildet sind. Auch Kombinationen von Steckbuchsen und Steckstiften an einem Steckverbinderteil sind möglich. Die beiden Steckelemente eines Steckverbinderteils sind gleich, d.h. zueinander parallel orientiert und legen somit eine gemeinsame Steckrichtung fest. Quer zu dieser Steckrichtung sind sie voneinander beabstandet. Sind mehr als zwei Steckelemente vorhanden, können diese in Querrichtung auf einer Linie angeordnet sein, so dass sie eine gerade Reihe bilden. Sie können von dieser Linie auch abweichen und somit z.B. im Dreieck, kreisförmig, auf einer Zickzacklinie, im Rechteck, Trapez, Quadrat, sternförmig oder anderweitig verteilt angeordnet sein. Die beiden Steckelemente können auch asymmetrisch ausgebildet sein. So kann sichergestellt werden, dass die Zuordnung von Stecker und Steckbuchse so eindeutig festgelegt wird, dass sie nur in einer einzigen Zuordnung und Ausrichtung zusammengefügt werden können.

Die Steckbuchse weist vorzugsweise eine zylindrische Buchsenöffnung auf, die einen Einsteckraum für einen Steckstift bildet. Der Einsteckraum kann an seinem offenen Ende trichterartig erweitert sein, um eine Einführhilfe zu bilden. Die Steckbuchse ist vorzugsweise, bis auf geringe Abweichungen, beispielsweise in Form von Fluidkanälen, Radialbohrungen und dergleichen, rotationssymmetrisch ausgebildet.

Die Steckbuchse kann aus einem Metall oder aus einem formstabilen Kunststoff gebildet sein. Ist die Steckbuchse aus Metall gebildet kann sie zur Verhinderung von Korrosion und von Reibung und damit zur Reduzierung von Steck- und Ziehkräften teilweise oder komplett mit einer Beschichtung beispielsweise einer PTFE Beschichtung versehen sein.

Der Steckstift ist vorzugsweise ebenfalls (bis auf geringe Abweichungen, beispielsweise in Form von Fluidkanälen) rotationssymmetrisch ausgebildet. Er weist dann im Wesentlichen eine zylindrische Mantelfläche und eine gegebenenfalls konische Stirnfläche auf. An seiner Mantelfläche ist es vorzugsweise mit zwei oder mehreren Dichtungselementen versehen, die voneinander axial beabstandet sind. Zwischen zwei Dichtungselementen kann an der Mantelfläche ein Fluidkanal münden, der dann mit einer zu- oder abführenden Fluidleitung kommuniziert.

Sind entsprechende Fluidsteckverbinderteile mit Steckstiften und Steckbuchsen zusammengefügt, stellen die Dichtelemente des Steckstifts eine fluiddichte Verbindung zwischen dem Steckstift und der Steckbuchse her. Die Dichtelemente sind in Radialrichtung elastisch, um ihre Dichtfunktion zu erbringen. Durch die zusätzliche Querbeweglichkeit wenigstens eines der Steckelemente, d.h. des Steckstifts und/oder der Steckbuchse, wird erreicht, dass der Steckstift und die Steckbuchse unabhängig von gegebenenfalls vorhandenen Maßtoleranzen sich beim Zusammenfügen konzentrisch zueinander ausrichten, so dass die zwischen Steckstift und Steckbuchse wirksamen Dichtelemente an ihrem gesamten Umfang gleichmäßig belastet sind. Damit wird eine Voraussetzung für eine dauerhaft gute Abdichtung solcher Steckverbindungen sowie für geringen Dichtungsverschleiß beim wiederholten Zusammenfügen und Trennen geschaffen. Außerdem kann unabhängig von Fertigungstoleranzen, Produktionschargen und Materialalterung, Temperaturunterschieden oder sonstigen Einflüssen, die zu minimalen Abweichungen der Steckelementabstände an Fluidsteckern oder Fluidsteckdosen führen können, erreicht werden, dass sich die Fluidstecker mit geringen Handkräften in die Fluidsteckdosen einführen und aus diesen herausziehen lassen.

Es lassen sich außerdem Fluidsteckverbinder gestalten, die eine große Anzahl von Einzelkanälen aufweisen. Es können Stecker und Buchsen miteinander in Eingriff gebracht werden, die nicht individuell gepaart, d.h. nicht speziell aufeinander abgestimmt sind. Der Verschleiß an den Dichtelementen insbesondere der Steckstifte ist minimiert. Die Steck- und Ziehkräfte sind auch bei ungepaarten Fluidsteckern und Fluidsteckdosen relativ gering. Auch kann sichergestellt werden, dass wegen der immer konzentrischen Ausrichtung von Steckstift und Steckbuchse zueinander immer gleiche Strömungsverhältnisse bestehen und es keine durch Fehljustagen sonst auftretende Strömungswirbel oder Kavitation gibt.

Wegen der verlässlich geringen Steck- und Ziehkräfte ist es auch möglich, dem Bediener eine taktile Rückmeldung über das ordnungsgemäße Einstecken des Steckers zu geben. Dazu kann beispielsweise eine Rastvorrichtung dienen. Das Einschnappen derselben wird deutlich fühlbar, weil die relativ geringen Steckkräfte, die beim Einschnappen der Rastvorrichtung auftretenden Kräfte nicht überdecken. Die Rastvorrichtung kann durch einen oder mehrere Schnapphaken und eine oder mehrere zugeordnete Rastausnehmung gebildet sein, die an den Fluidsteckverbinderteilen ausgebildet sind.

An dem Stecker kann außerdem eine Abdeckkappe beweglich gelagert sein. Diese kann quer beweglich gelagerte Buchsen zum Verschluss der Steckstifte aufweisen. Alternativ oder zusätzlich können die Steckstifte eine gewisse Querbeweglichkeit aufweisen. In diesem Fall können die Aufnahmeöffnungen der Abdeckkappe zur Aufnahme der Steckstifte an der Abdeckkappe starr (also unbeweglich) angeordnet sein. Eine Abdeckkappe ist meist bei der Wiederaufbereitung und Sterilisation von medizinischen Instrumenten notwendig. Auch die Abdeckkappe kann über einen Schnappmechanismus mit dem Stecker verbunden sein.

Zur Erbringung wenigstens einiger der oben genannten Vorteile kann es genügen, wenn nur einige der Steckbuchsen beweglich gelagert sind, beispielsweise zwei von drei. Es wird aber als vorteilhaft angesehen, wenn alle Steckbuchsen des betreffenden Fluidsteckverbinderteils in mindestens einer Querrichtung beweglich gelagert sind. Beispielsweise können alle Steckbuchsen aufeinander zu und voneinander weg beweglich gelagert sein. Es ist auch möglich, eine Steckbuchse in einer ersten und eine andere Steckbuchse in einer zweiten von der ersten Querrichtung verschiedenen Querrichtung beweglich zu lagern. Auch damit können wenigstens einige der oben genannten Vorteile erbracht werden.

Vorzugsweise sind wenigstens einige der Steckbuchsen, vorzugsweise alle, aufeinander zu und voneinander weg beweglich. Es ist auch möglich, ein, einige oder alle Steckbuchsen in allen Radialrichtungen beweglich zu lagern.

Es ist möglich, die Steckbuchsen frei beweglich, d.h. mit einem gewissen seitlichen Spiel zu lagern. Es wird jedoch bevorzugt, Federmittel vorzusehen, die dazu dienen, die Steckbuchse in einer Ruheposition zu halten. Diese Ruheposition kann eine Mittelposition sein. Zur federnden Lagerung können ein oder mehrere Federelemente, beispielsweise in Gestalt von schlauch- oder ringförmigen Elastomerelementen dienen. Die Elastomerelemente können vorgefertigte Elemente mit porenlosen oder geschäumten Körpern sein. Es kann sich auch um Elastomerelemente handeln, die durch Eindringen einer formlosen Masse in entsprechende Räume des Fluidsteckverbinderteils und Aushärten der Masse geschaffen sind. Die Elastomerelemente können einen Vollkörper oder einen mit mindestens einem Hohlraum versehenen Körper aufweisen. Sie können als O-Ringe, zu einem Ring gebogene Elastomerschlauchabschnitte, in einer Schraubenlinie gelegte Schlauchabschnitte oder beliebige andere Federmittel ausgebildet sein.

Die beweglich gelagerten Steckbuchsen sind vorzugsweise von einer mit ihnen gemeinsam beweglich gelagerten Gasführungshülse umgeben. Das Federelement stützt vorzugsweise die genannte Gasführungshülse. Zwischen der Gasführungshülse und der Steckbuchse sind vorzugsweise Dichtelemente angeordnet, die die Gasführungshülse und die Steckbuchse zueinander konzentrisch halten und einen Gasführungsraum abgrenzen. Der Gasführungsraum ist vorzugsweise ein Ringraum bzw. ein ringspaltförmiger Raum. Nach außen ist er dicht. Außerdem ist er vorzugsweise über mindestens einen Kanal mit einer von dem Fluidsteckverbinderteil wegführenden (oder zu ihm hinführenden) Leitung verbunden. Außerdem ist der Gasführungsraum über eine oder mehrere Radialbohrungen mit der Buchsenöffnung verbunden.

Die Federkonstante der zwischen der Steckbuchse und der Gasführungshülse wirksamen Dichtelemente ist vorzugsweise größer als die Federkonstante der Federelemente. Entsprechend ist die Nachgiebigkeit der Federelemente deutlich größer als die Nachgiebigkeit der Dichtelemente. Dadurch werden beim Einstecken des Steckstifts in die Steckbuchse und bei einer dabei auftretenden seitlichen Verlagerung der Steckbuchse die Federmittel, nicht aber die Dichtungen verformt.

Weitere Einzelheiten vorteilhafter Ausführungsformen der Erfindung sind Gegenstand von Ansprüchen, der Zeichnung oder der Beschreibung. Es zeigen:
Fig. 1 einen Fluidsteckverbinder mit zwei Fluidsteckverbinderteilen, in schematischer Gesamtdarstellung;
Fig. 2 ein als Buchsenteil ausgebildetes Steckverbinderteil, in schematisierter ausschnittsweiser Vertikalschnittdarstellung;
Fig. 3 ein als Stecker ausgebildetes Fluidsteckverbinderteil mit Abdeckkappe, die unverlierbar mit dem Stecker verbunden ist, in teilweise geschnittener Draufsicht;
Fig. 4 den Stecker nach Fig. 3, in Seitenansicht;
Fig. 5 und 6 Beispiele eines als Stecker ausgebildeten Fluidverbinderteils in jeweils ausschnittsweiser, teilweise geschnittener Draufsicht.

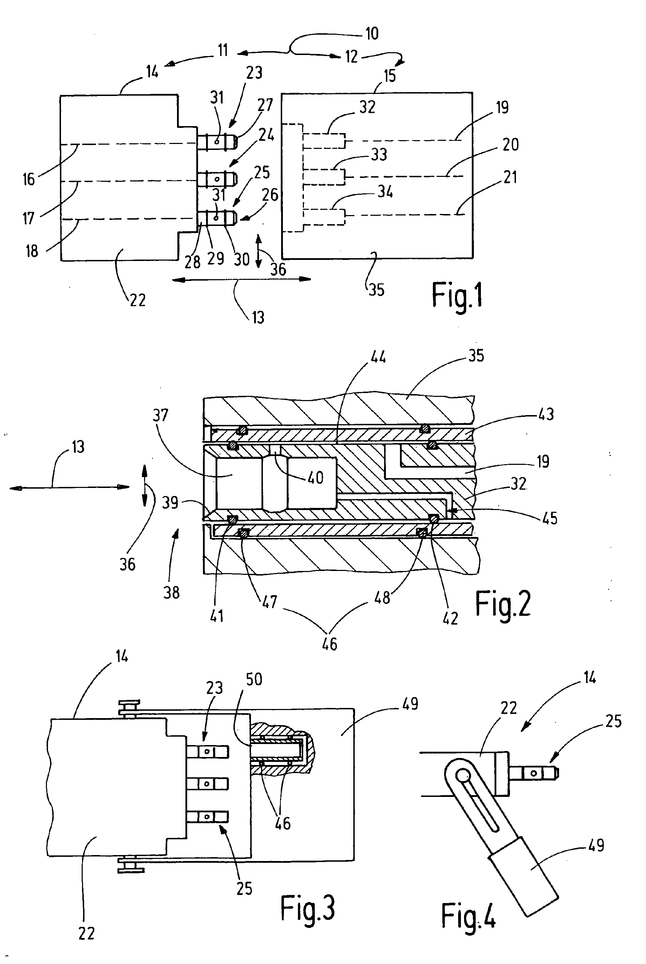

In Fig. 1 ist ein Fluidsteckverbinder 10 veranschaulicht, zu dem zwei Steckverbinderteile 11, 12 gehören, die in einer Steckrichtung 13 zusammenführbar und auseinanderziehbar sind. Das Steckverbinderteil 11 ist als Stecker 14 ausgebildet. Das Steckverbinderteil 12 ist als Fluidsteckdose 15 ausgebildet.

Beispielsweise gehört der Fluidstecker 14 zu einem medizinischen Instrument, beispielsweise einem kryotechnischen Instrument, das im vorliegenden Fall über insgesamt drei Fluidkanäle 16, 17, 18 verfügt, die zur Zu- und Abführung von flüssigen oder gasförmigen Fluiden dienen. Die Fluidkanäle 16 bis 18 setzen sich über rohr- oder schlauchartige Leitungen zu dem distalen Ende des Instruments fort, was in Fig. 1 nicht weiter dargestellt ist.

Die Fluidkanäle 16 bis 18 kommunizieren im Betriebszustand mit Fluidkanälen 19, 20, 21, die in einem nicht weiter veranschaulichten medizinischen Gerät mit Quellen oder Senken für die Fluide verbunden sind und zu der Fluidsteckdose 15 führen.

Der Fluidstecker 14 weist ein Steckergehäuse 22 auf, von dem sich zwei oder mehrere, beispielsweise drei Steckstifte 23, 24, 25 in Steckrichtung 13 weg erstrecken. Die Steckstifte 23 bis 25 können, wie es bevorzugt wird, untereinander gleich ausgebildet sein. Sie können jedoch auch unterschiedliche Dimensionen, beispielsweise unterschiedliche Längen und/oder unterschiedliche Querschnitte oder Durchmesser haben, um ein Zusammenfügen des Fluidsteckers 14 mit der Fluidsteckdose 15 nur in einer ausgewählten einzigen Position zu ermöglichen. Die Steckstifte 23 bis 25 können auf einer geraden Linie in gleichen Abständen zueinander oder, um Verwechslungen zu vermeiden, in ungleichen Abständen oder auch auf einer nicht geraden Linie, z.B. im Dreieck oder auf einem Kreisbogen, angeordnet sein. Die Querschnitte der Steckstifte 23 bis 25 sind vorzugsweise Kreisquerschnitte. Es können auch abweichende Querschnittsformen Anwendung finden.

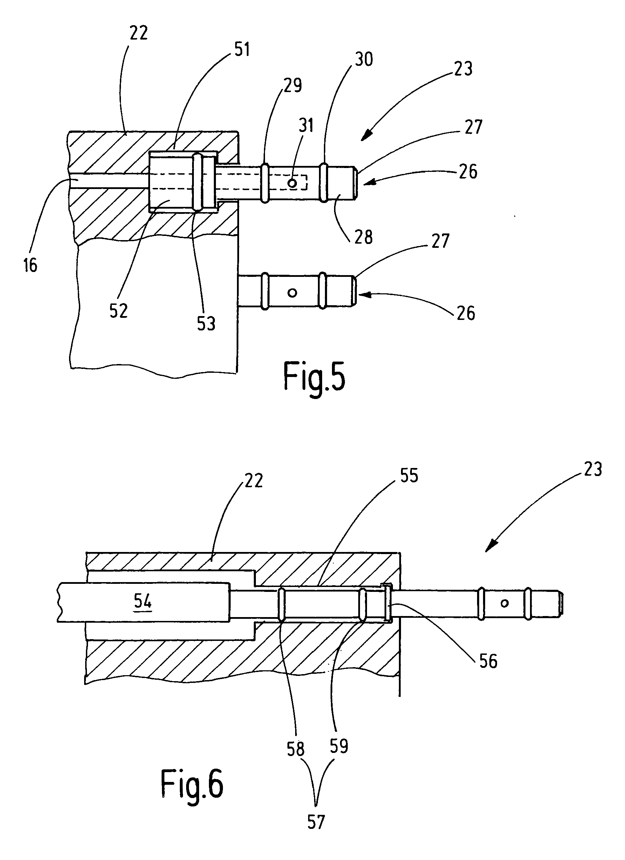

Unabhängig von Größe und Dimensionierung weist jeder Steckstift 23 bis 25 einen Grundkörper auf, der vorzugsweise im Wesentlichen kreiszylindrisch ist. An seiner Stirn kann eine Einführhilfe 26 vorgesehen sein, beispielsweise in Form einer konischen Fläche 27 (siehe auch Fig. 5). Weiter weist jeder Steckstift 23 bis 25 an seiner vorzugsweise zylindrischen Umfangsfläche 28 zwei Dichtungselemente 29, 30 vorzugsweise in Gestalt von Dichtringen, z.B. O-Ringen auf, zwischen denen eine Fluidöffnungen 31 ausgebildet ist, die mit dem zugeordneten Fluidkanal 16 kommuniziert. Dies gilt entsprechend für alle anderen Steckstifte 23, 24 und die Fluidkanäle 17, 18 in gleicher Weise.

Den Steckstiften 23 bis 25 sind Steckbuchsen 32 bis 34 zugeordnet, die in Fig. 1 gestrichelt veranschaulicht sind. Die Steckstifte 23 bis 25 und die Steckbuchsen 32 bis 34 werden hier allgemein als "Steckelemente" bezeichnet.

Fig. 2 veranschaulicht die Steckbuchse 32 exemplarisch und stellvertretend für die anderen beiden Steckbuchsen 33, 34. Mindestens eine, besser zwei, vorzugsweise aber alle Steckbuchsen 32 bis 34 sind in einem Steckverbinderkörper einem Gehäuse 35 der Fluidsteckdose 15 in einer Querrichtung 36 beweglich gelagert. Es kann auch von einer "schwimmenden" Lagerung gesprochen werden. Dazu weist die Steckbuchse 32 eine begrenzte Beweglichkeit in einer ersten Querrichtung 36 auf, die quer zu der Steckrichtung 13 gerichtet ist und die zu der benachbarten Steckbuchse 33 hin und/oder von dieser weggerichtet ist. Zusätzlich oder alternativ können mindestens eine, mehrere oder alle der Steckbuchsen 32 bis 34 in einer anderen Querrichtung, z.B. in einer beliebigen anderen Radialrichtung beweglich gelagert sein. Im vorliegenden Ausführungsbeispiel sind die Steckbuchsen 32 bis 34 in allen Radialrichtungen gleichmäßig beweglich gelagert.

Die Steckbuchse 32 weist eine Buchsenöffnung 37 auf, die im Wesentlichen durch eine Sackbohrung gebildet ist. Der Querschnitt jeder Buchsenöffnung 37 ist auf den Querschnitt des jeweils zugeordneten Steckstifts 23 - 25 passend abgestimmt. An ihrem offenen Ende kann die Buchsenöffnung 37 mit einer Einführhilfe 38 in Gestalt einer konischen Fläche 39 versehen sein. Ansonsten ist die Buchsenöffnung 37 vorzugsweise im Wesentlichen zylindrisch. An einer Stelle, die bei eingestecktem Steckstift 23 auf gleicher Höhe mit der Fluidöffnung 31 liegt, können eine oder mehrere Radialbohrungen 40 vorgesehen sein.

An dem vorzugsweise zylindrischen Außenumfang der Steckbuchse 32 sind zwei axial voneinander beabstandete Dichtungselemente 41, 42, vorzugsweise Dichtungsringe, z.B. O-Ringe angeordnet, die z.B. in axial voneinander beabstandeten Ringnuten der Steckbuchse 32 sitzen. Zwischen den Dichtungselementen 41, 42 zweigt der Fluidkanal 19 ab, der an der Umfangsfläche der Steckbuchse 32 mündet.

Die Steckbuchse 32 ist von einer Gasführungshülse 43 aufgenommen, die z.B. als kurzes gerades Rohrstück ausgebildet sein kann. Mit ihrer Innenumfangsfläche liegt sie an den Dichtungselementen 41, 42 der Steckbuchse 32, beispielsweise den O-Ringen, an. Sie begrenzt mit der Steckbuchse 32 einen Gasführungsraum 44, der mit dem Fluidkanal 19 und der Radialbohrung 40 kommuniziert. Ein weiterer Kanal 45 mündet bodenseitig in der Buchsenöffnung 37 und verbindet diese mit dem zwischen der Steckbuchse 32 und der Gasführungshülse 43 ausgebildeten Ringspalt jedoch außerhalb des Gasführungsraums 44. Dieser Kanal 45 dient der Abführung verdrängter Fluide, beispielsweise Luft, beim Einstecken des Steckstifts 23 in die Buchsenöffnung 37.

Die aus der Steckbuchse 32 und der Gasführungshülse 43 gebildete Einheit ist in dem Gehäuse 35 in einer entsprechenden Aufnahme, zumindest in der einen Querrichtung 36, vorzugsweise aber insgesamt radial z.B. mit Spiel beweglich angeordnet. Zur Vermeidung einer undefinierten Lage und somit zur nachgiebigen Zentrierung der Steckbuchse 32 kann sich die Gasführungshülse 43 auch über mindestens ein oder auch mehrere Federelemente 46 an dem Gehäuse 35 abstützen. Die Federelemente 46 können Metallfedern, beispielsweise in Gestalt von gewellten, ringförmig geschlossenen oder geschlitzten (C-förmigen) Blechringen oder auch Kunststoffelemente, beispielsweise in Gestalt von Ringen, Schnüren oder dergleichen sein. Es kann geschäumtes oder solides Material zum Einsatz kommen. Im vorliegenden Ausführungsbeispiel sind als Federelemente 46 zwei O-Ringe 47, 48 vorgesehen. Es können jedoch auch Federelemente 46, insbesondere O-Ringe, in abweichender Zahl Verwendung finden. Die Federelemente 46 sind in ihrer Federwirkung nachgiebiger ausgebildet als die z.B. durch die O-Ringe gebildeten Dichtungselemente 41, 42. Auf diese Weise bewirkt eine seitliche Verlagerung der Steckbuchse 32 vorzugsweise vorwiegend eine Verformung des mindestens einen Federelements 46, wobei die Gasführungshülse 43 und die Steckbuchse 32 vorzugsweise zueinander im Wesentlichen zentriert bleiben. Auf diese Weise bleibt auch der Gasführungsraum 44 vorzugsweise im Wesentlichen unverformt, d.h. die Spaltweite dieses ringspaltförmigen Raums wird nicht nachteilig geändert.

Der insoweit beschriebene Fluidsteckverbinder 10 arbeitet wie folgt:

Zur Beschreibung der Funktion wird davon ausgegangen, dass die Steckstifte 23 bis 25 zwar im Wesentlichen, jedoch nicht exakt mit ihren Steckbuchsen 32 bis 34 fluchten. Wird der Fluidstecker 14 nun in die Fluidsteckdose 15 eingeführt, zentrieren sich die radial beweglichen Steckbuchsen 32, 33, 34, indem die konischen Flächen 27 der Steckstifte 23 bis 25 mit den entsprechenden konischen Flächen 39 der Steckbuchsen 32 bis 34 zusammen wirken und dadurch die jeweilige Steckbuchse 32 bis 34 radial bewegen. Dabei werden die Federelemente 46 entsprechend deformiert. Diese sind ausreichend nachgiebig und geben mit geringer Kraft nach. Die Steckstifte 23 bis 25 können so mit geringer Kraft in die Steckbuchsen 32 bis 34 eingeschoben werden. Die Dichtungen 29, 30 zentrieren dabei den Steckstift 23 in der Steckbuchse 32. Die Dichtungen 29, 30 werden dabei entlang ihres Umfangs im Wesentlichen gleichmäßig verformt. Entsprechendes gilt für die übrigen Paarungen. Die Dichtungen 29, 30 sind erheblich weniger nachgiebig als die Federelemente 46. Wie schon die Dichtungen 41, 42, sind auch die Dichtungen 29, 30 entlang ihres Umfangs im Wesentlichen gleichmäßig verformt. Es ist deshalb eine entlang des gesamten Umfangs gleichmäßig gute und prozesssichere Abdichtung zwischen dem Steckstift 23 und der inneren Wandung der Buchsenöffnung 37 gegeben. Auch ist und bleibt der Gasführungsraum 44 prozesssicher gut abgedichtet.

In eingestecktem Zustand fluchtet vorzugsweise die Fluidöffnung 31 mit der Radialbohrung 40. Somit sind die Fluidkanäle 16, 19 miteinander in nach außen abgedichteter Kommunikation. Es können auch unter hohem Druck flüssige oder gasförmige Fluide ungestört zwischen den Fluidkanälen 16, 19 ausgetauscht werden.

Wie die Figuren 3 und 4 veranschaulichen, kann der Fluidstecker 14 z.B. zu Aufbewahrungszwecken oder zu Reinigungszwecken mit einer Verschlusskappe 49 versehen sein, die an dem Steckergehäuse 22 z.B. verliersicher gehalten sein kann. Eine solche Verschlusskappe 49 kann als Steckbuchsen dienende Blindbuchsen 50 enthalten, wie in Fig. 3 lediglich anhand des dem Steckstift 23 zugeordneten Steckplatzes veranschaulicht ist. Die Blindbuchse 50 kann unmittelbar durch die Federmittel 46 an dem Körper der Verschlusskappe 49 abgestützt sein. Wiederum ist die seitliche Beweglichkeit der Blindbuchsen 50 dazu geeignet, etwaige Abstandstoleranzen oder sonstige Anordnungstoleranzen der Steckstifte 23 bis 25 auszugleichen und so ein leichtes Aufstecken der Abdeckkappe 49 zu ermöglichen, ohne dabei die Dichtungen 29, 30 zu beschädigen oder zu überanspruchen.

Bei der vorigen Beschreibung ist davon ausgegangen worden, dass zumindest zwei der drei Steckbuchsen 32 bis 34 radial beweglich angeordnet sind, wobei die radiale Beweglichkeit durch eine entsprechend zentrierende Federkraft des mindestens einen Federelements 46 nicht eingeschränkt wird. Die von dem(den) Federelement(en) 46 verursachte Federkraft ist geringer als die von den Dichtungen 29, 30 oder den Dichtungen 41, 42 erzeugte Federkraft. Dabei können die Steckstifte 23 bis 25 an dem Steckergehäuse 22 starr angeordnet sein. Es ist jedoch alternativ oder ergänzend möglich, wenigstens einen, mehrere oder alle der Steckerstifte 23 bis 25 radial beweglich zu lagern. Beispiele dafür sind in den Figuren 5 und 6 angegeben.

Das Steckergehäuse 22 kann eine Kammer 51 zur Aufnahme eines Endes 53 des Steckstifts 23 aufweisen. Das Steckergehäuse 22 kann eine nicht dargestellte Trennfuge aufweisen, die durch die Kammer 51 verläuft und die Montage des Steckstifts 23 ermöglicht. Dieser kann in der Kammer 51 mit einer Dichtung 53 abgedichtet gehalten sein. Die Dichtung 53 dient hier zugleich als Federmittel. Sie kann so weich ausgebildet sein, dass sie einer Radialbewegung des Steckstifts 23 eine Kraft entgegensetzt, die wesentlich geringer ist als eine entsprechende, bei gleicher Deformation von den Dichtungen 29, 30 verursachte Kraft. Die Kammer 51 kommuniziert mit dem Fluidkanal 16 der, wie in Fig. 5 gestrichelt veranschaulicht, sich axial durch den Steckstift 23 bis zu der Fluidöffnung 31 hin fortsetzt.

Ein anderes eispiel nach Fig. 6 nutzt den Fluidstecker 23 als direkten Anschluss für einen Fluidschlauch 54, der durch geeignete, nicht weiter veranschaulichte Fluidverbindungsmittel unmittelbar mit dem proximalen Ende des Steckstifts 23 verbunden ist. Der Steckstift 23 erstreckt sich mit radialem Spiel durch eine Öffnung 55 des Steckergehäuses 22. Zur axialen Abstützung kann der Steckstift 23 mit einem Bund 56, der mit Spiel in einer entsprechenden Ringnut der Öffnung 55 sitzt, oder anderem geeigneten Axialbefestigungsmittel versehen sein. An dem Steckstift 23 können Federmittel 57 angebracht sein, beispielsweise in Gestalt eines oder zweier Elastomerringe 58, 59. Diese Elastomerringe 58, 59 können beispielsweise durch O-Ringe, vorzugsweise weiche O-Ringe gebildet sein. Alternativ können andere Federmittel zum Einsatz kommen.

Es kann auf die Federmittel 57 auch verzichtet werden. Außerdem können sie durch Strukturen des Steckergehäuses 22 ersetzt sein, wie beispielsweise durch einzelne oder mehrere Kunststoffzungen, eine zunächst formlose und dann aushärtende Elastomermasse oder ähnliches.

Ein Fluidsteckverbinder weist Steckelemente, beispielsweise in Gestalt von Steckstiften 23 bis 25 oder Steckbuchsen 32 bis 34 auf, die quer zu einer Steckrichtung 13 wenigstens geringfügig beweglich gelagert sind. Federmittel 46, 57 können zur Vorgabe einer Ruheposition der entsprechenden Steckelemente dienen, um sie beweglich und spielfrei anzuordnen. Dadurch wird ein Klappern verhindert und/oder das Entstehen einer sichtbaren, unregelmäßig, nicht konzentrischen Anordnung einer Steckbuchse 32 zu einer Bohrung in dem Gehäuse 35. Diese nicht konzentrische Anordnung könnte als optischer, bzw. qualitativer Mangel empfunden werden. Bezugszeichenliste:
- 10: Fluidsteckverbinder
- 11, 12: Steckverbinderteil
- 13: Steckrichtung
- 14: Fluidstecker, umfasst einen oder mehrere Steckstifte 23 - 25
- 15: Fluidsteckdose, umfasst eine oder mehrere Steckbuchsen 32 - 34
- 16 - 18: Fluidkanal
- 19 - 21: Fluidkanal
- 22: Steckergehäuse
- 23 - 25: Steckstift
- 26: Einführhilfe
- 27: Fläche
- 28: Umfangsfläche
- 29, 30: Dichtungen
- 31: Fluidöffnung
- 32 - 34: Steckbuchse
- 35: Steckverbinderkörper, Gehäuse
- 36: Querrichtung
- 37: Buchsenöffnung
- 38: Einführhilfe
- 39: Fläche
- 40: Radialbohrung
- 41, 42: Dichtungselement, O-Ring
- 43: Gasführungshülse
- 44: Gasführungsraum
- 45: Kanal
- 46: Federelement
- 47, 48: O-Ring
- 49: Verschlusskappe
- 50: Blindbuchse
- 51: Kammer
- 52: Ende
- 53: Dichtung
- 54: Schlauch
- 55: Öffnung
- 56: Bund
- 57: Federmittel
- 58, 59: Elastomerring

## Patentansprüche

1. Fluidsteckverbinderteil (12) insbesondere für medizinische Geräte oder Instrumente;
das mindestens zwei Steckbuchsen (32 - 34) aufweist, die eine gemeinsame Steckrichtung (13) festlegen,
wobei wenigstens eine der Steckbuchsen (32 - 34) in mindestens einer Querrichtung (36) beweglich gelagert ist, die zu der Steckrichtung (13) quer orientiert ist,
**dadurch gekennzeichnet,**
**dass** die beweglich gelagerte Steckbuchse (32 - 34) von einer beweglich gelagerten Gasführungshülse (43) umgeben ist,
**dass** die Gasführungshülse (43) durch mehrere voneinander beabstandete Federelemente (46) an einem Steckverbinderkörper (35) abgestützt ist, die eine erste Federkonstante aufweisen,
**dass** die Steckbuchse (32 - 34) mit zwei voneinander beabstandeten Dichtungselementen (41, 42) in der Gasführungshülse (43) einen Gasführungsraum (44) abgrenzt, wobei die Dichtungselemente (41, 42) eine zweite Federkonstante aufweisen, die größer ist als die erste Federkonstante, und
**dass** die Steckbuchse (32 - 34) mindestens eine Radialbohrung (40) aufweist, die den Gasführungsraum (44) mit einer Buchsenöffnung (37) verbindet.

2. Fluidsteckverbinderteil nach einem der vorstehenden Ansprüche, **dadurch gekennzeichnet, dass** alle Steckbuchsen (32 - 34) in der mindestens einen Querrichtung (36) beweglich gelagert sind.

3. Fluidsteckverbinderteil nach einem der vorstehenden Ansprüche, **dadurch gekennzeichnet, dass** die Steckbuchsen (32 - 34) in der Querrichtung (36) aufeinander zu und voneinander weg beweglich gelagert sind.

4. Fluidsteckverbinderteil nach einem der vorstehenden Ansprüche, **dadurch gekennzeichnet, dass** mindestens eine der Steckbuchsen (32 - 34) in allen radial zu der Steckrichtung (13) orientierten Richtungen beweglich ist.

5. Fluidsteckverbinderteil nach einem der vorstehenden Ansprüche, **dadurch gekennzeichnet, dass** die Steckbuchsen (32 - 34) in einer Reihe angeordnet sind.

6. Fluidsteckverbinderteil nach Anspruch 1, **dadurch gekennzeichnet, dass** die Federelemente (46) ringförmige Elastomerkörper sind.

7. Fluidsteckverbinderteil nach einem der vorstehenden Ansprüche, **dadurch gekennzeichnet, dass** Steckelementbuchsen (32 - 34) an ihrer Stirnseite eine Einführhilfe (38) aufweist.

8. Fluidsteckverbinderteil nach einem der vorstehenden Ansprüche, **dadurch gekennzeichnet, dass** die Einführhilfe (38) durch eine Konusfläche (27, 39) gebildet ist.

## Claims

1. Fluid plug connector part (12), in particular for medical devices or instruments;
which has at least two sockets (32 - 34) which define a mutual plug direction (13),
wherein at least one of the sockets (32 - 34) is mounted to be moveable in at least one transverse direction (36) which is orientated transversely to the plug direction (13),
**characterised in that**
the moveably mounted socket (32 - 34) is surrounded by a moveably mounted gas guiding sleeve (43),
the gas guiding sleeve (43) is supported on a plug connector body (35) by several spring elements (46) which are at a distance to one another, said spring elements having a first spring constant,
the socket (32 - 34) delimits a gas guiding chamber (44) with two sealing elements (41, 42) which are at a distance to each other in the gas guiding sleeve (43), wherein the sealing elements (41, 42) have a second spring constant which is greater than the first spring constant,
and the socket (32 - 34) has at least one radial bore (40) which connects the gas guiding chamber (44) to a socket opening (37).

2. Fluid plug connector part according to one of the preceding claims, **characterised in that** all sockets (32 - 34) are mounted to be moveable in the at least one transverse direction (36).

3. Fluid plug connector part according to one of the preceding claims, **characterised in that** the sockets (32 - 34) are mounted to be moveable towards one another and away from one another in the transverse direction (36).

4. Fluid plug connector part according to one of the preceding claims, **characterised in that** at least one of the sockets (32 - 34) is moveable in all directions orientated radially to the plug direction (13).

5. Fluid plug connector part according to one of the preceding claims, **characterised in that** the sockets (32-34) are arranged in a row.

6. Fluid plug connector part according to claim 1, **characterised in that** the spring elements (46) are annular elastomer bodies.

7. Fluid plug connector part according to one of the preceding claims, **characterised in that** the sockets (32-34) have an insertion aid (38) on their front side.

8. Fluid plug connector part according to one of the preceding claims, **characterised in that** the insertion aid (38) is formed by a conical surface (27, 39).

## Revendications

1. Connecteur à fiches fluidique (12), destiné en particulier à des appareils ou instruments médicaux,
qui présente au moins deux fiches femelles (32 à 34) qui définissent un sens d'enfichage (13) commun,
sachant qu'au moins une des fiches femelles (32 à 34) est montée mobile dans au moins une direction transversale (36) qui est orientée transversalement au sens d'enfichage (13),
**caractérisé en ce que**
la fiche femelle (32 à 34) montée mobile est entourée d'un manchon de guidage de gaz (43) monté mobile,
**en ce que** le manchon de guidage de gaz (43) est en appui sur un corps de connecteur à fiches (35) par l'intermédiaire de plusieurs éléments élastiques (46) espacés les uns des autres, qui présentent une première constante de ressort,
**en ce que** la fiche femelle (32 à 34) délimite, avec deux éléments d'étanchéité (41, 42) espacés l'un de l'autre, une chambre de guidage de gaz (44) dans le manchon de guidage de gaz (43), sachant que les éléments d'étanchéité (41, 42) présentent une deuxième constante de ressort qui est supérieure à la première constante de ressort,
et **en ce que** la fiche femelle (32 à 34) présente au moins un trou radial (40) qui relie la chambre de guidage de gaz (44) à un orifice de fiche femelle (37).

2. Connecteur à fiches fluidique selon l'une des revendications précédentes, **caractérisé en ce que** toutes les fiches femelles (32 à 34) sont montées mobiles dans la direction transversale (36), au nombre d'au moins une.

3. Connecteur à fiches fluidique selon l'une des revendications précédentes, **caractérisé en ce que** les fiches femelles (32 à 34) sont montées de manière à pouvoir être rapprochées et éloignées les unes des autres dans la direction transversale (36).

4. Connecteur à fiches fluidique selon l'une des revendications précédentes, **caractérisé en ce qu'**au moins une des fiches femelles (32 à 34) est mobile dans toutes les directions orientées radialement par rapport au sens d'enfichage (13).

5. Connecteur à fiches fluidique selon l'une des revendications précédentes, **caractérisé en ce que** les fiches femelles (32 à 34) sont disposées dans une rangée.

6. Connecteur à fiches fluidique selon la revendication 1, **caractérisé en ce que** les éléments élastiques (46) sont des corps élastomères annulaires.

7. Connecteur à fiches fluidique selon l'une des revendications précédentes, **caractérisé en ce que** les fiches femelles (32 à 34) présentent une aide d'insertion (38) sur leur face frontale.

8. Connecteur à fiches fluidique selon l'une des revendications précédentes, **caractérisé en ce que** l'aide d'insertion (38) est constituée d'une surface conique (27, 39).
